# EUROPEAN PATENT SPECIFICATION

(11) **EP 0 542 366 B1**
(45) Date of publication and mention of the grant of the patent: **25.02.1998**
(21) Application number: 92203452.5
(22) Date of filing: 10.11.1992
(51) Int. Cl.: C07C 29/36, C07C 209/60, C07B 41/02, C07B 43/04, C07B 61/00

(54) **Process for the telomerization of conjugated alkadienes**
Verfahren zur Telomerisation von konjugierten Alkadienen
Procédé pour la telomérisation d'alkadienes conjugués

(30) Priority: 12.11.1991 EP 91202941
(43) Date of publication of application: 19.05.1993
(73) Proprietor: SHELL INTERNATIONALE RESEARCH MAATSCHAPPIJ B.V., 2596 HR Den Haag (NL)
(72) Inventor: Drent, Eit, NL-1031 CM Amsterdam (NL)

(56) References cited:
- EP-A- 0 361 304
- GB-A- 1 256 357
- GB-A- 2 074 156
- US-A- 3 530 187
- US-A- 4 120 901
- BULLETIN OF THE CHEMICAL SOCIETY OF JAPAN vol. 45, no. 4, April 1972, TOKYO JP pages 1183 - 1191 K. TAKAHASHI ET AL 'PALLADIUM-CATALYZED REACTIONS OF 1,3-DIENES WITH ACTIVE METHYLENE COMPOUNDS. IV. PALLADIUM-DIPHOSPHINE COMPLEX CATALYSTS'
- TETRAHEDRON LETTERS. vol. 33, no. 14, 31 March 1992, OXFORD GB pages 1831 - 1834 B. M. TROST ET AL 'ATOM ECONOMY. A SIMPLE Pd CATALYZED ADDITION OF PRONUCLEOPHILES WITH DIENES'

## Description

The present invention relates to a process for the telomerization of conjugated dienes, and more in particular to such a process conducted in the presence of a catalyst based on a palladium compound and a phosphine ligand, and to the products thus prepared.

Telomerization is a known reaction and refers to the reaction of conjugated alkadienes in the presence of a compound having an active hydrogen, such as for example a hydroxy compound, a primary or secondary amine, a carboxylic acid, a compound containing an active methylene group, and water.

The telomerization of conjugated alkadienes is known for example from Dutch Patent Specification 156 387, equivalent of British Patent Specification 1 256 357, which document relates to the oligomerization of specified conjugated dienes in the presence of a compound containing active hydrogen and a catalyst based on a combination of a bivalent palladium compound and a phosphine or arsine. Although both mono- and diphosphines and arsines are claimed, there appears to be a strong preference for the use of monophosphines, and especially triphenylphosphines. In fact in all the examples the catalyst either is based on a monophosphine or a monoarsine. From the large number of examples provided it can be observed that an acceptable degree of alkadiene-conversion and/or the selectivity to the desired reaction product is only obtained when the active hydrogen containing compound employed is an organic hydroxy compound. When using other types of active hydrogen containing compounds the alkadiene conversion and selectivity leave considerable room for improvement.

Bull. Chem. Soc. Japan 45 (1972), No. 4, pp 1183-1191 disclose the conjugation reaction of 1,3-dienes with active methylene compounds such as acetylacetone, ethyl acetoacetate and primary and secondary amines, in the presence of a catalyst system comprising a diphosphine/palladium complex. In most cases the product of the reaction is an adduct of one diene with one active methylene molecule. In only two cases is a telomer produced, and then in minor amounts.

As mentioned hereinbefore water is also considered to be an active hydrogen containing compound for use in telomerization reactions, as it offers the possibility of converting the alkadienes to the corresponding hydroxy dimercompounds, which compounds may be used e.g. in organic synthesis. The only information provided in said Dutch Patent Specification regarding the use of water is as a solvent.

The preparation of 2.7-octadienol-1 via a catalytic telomerization of butadiene in the presence of water is known from Russian Patent Application SU 979-316-A. Said method however, has a disadvantage in that it employs a rather complicated multicomponent catalyst system which comprises a specified metal sulphate, a palladium salt, a phosphine (such as an alkyl or aryl monophosphine) or a phospine and an organo-aluminium reducing agent.

It will be appreciated that the known catalyst system based on a palladium compound and a monophosphine such as for example triphenylphosphine, will be less stable and thus have a shorter life time, than similar catalyst systems containing chelating ligands such as for example 1.2-bis(diphenylphosphino)ethane.

Hence it can be concluded that there is considerable need for improvement in the telomerization of alkadienes. The problem underlying the present invention is to develop a process for the telomerization of alkadienes which does not suffer from one or more of the disadvantages as described hereinbefore. Thus, there is a need to develop a process for the telomerization of alkadienes which is conducted in the presence of active hydrogen-containing compounds, which combines a high alkadiene conversion with a high selectivity and/or which can be conducted in the presence of a relatively simple catalyst system.

As a result of extensive research and experimentation it was now found that the telomerization of alkadienes in the presence of certain active hydrogen-containing compounds could conveniently be conducted in the presence of a catalyst based on a palladium compound and selected diphosphines.

Accordingly the invention provides a process for the telomerization of conjugated alkadienes which comprises contacting a conjugated alkadiene with a compound having at least one active hydrogen atom selected from the group consisting of water and primary or secondary amines, in the presence of a catalyst obtainable from a palladium compound and a diphosphine of general formula R¹R²PRPR³R⁴, wherein R is a bivalent organic bridging group containing at least 3 atoms in the bridge, of which at least two are carbon atoms; R¹,R²,R³ and R⁴ are the same or different and represent optionally polar substituted hydrocarbyl groups, with the proviso that when the compound having active hydrogen is water the reaction is conducted in the presence of carbon dioxide and when the compound having active hydrogen is a primary or secondary amine the diphosphine is a bis(dialkylphosphino) based diphosphine and preferably a bis(di-n-butylphosphino) or a bis(diisopropylphosphino) based diphosphine.

Preferably the bridging group R of the diphosphine mentioned hereinbefore contains 3 to 5 atoms in the bridge. Examples of suitable bridging groups R include -(CH₂)₃-, -(CH₂)₄-, -CH₂-C(CH₃)₂-CH₂ and -CH₂-Si(CH₃)₂-CH₂-.

The hydrocarbyl groups R¹,R²,R³ and R⁴ are preferably the same and represent an aryl group, a phenyl group being the preferred aryl group, or an alkyl group having up to 10 carbon atoms. Preferred said alkyl groups have from 2 to 5 carbon atoms and may be linear or branched as exemplified by ethyl, propyl, isopropyl, butyl, tert-butyl and pentyl. Preferred diphosphines for use in the process of the present invention are 1,3-bis(diphenylphosphino)-propane, 1,4-bis(diphenylphosphino)butane, 1,3-bis(diethylphosphino)propane, 1,3-bis(diisopropylphosphino)propane, 1,3-bis(di-n-butylphosphino)propane and 1,4-bis(di-n-butylphosphino)butane.

The palladium compound on which the catalyst in the process of the present invention is based is preferably a palladium salt of a carboxylic acid, and palladium acetate in particular.

The conjugated alkadienes which may be converted by the process of the present invention will generally have from 4 to 8 carbon atoms and include 1,3-butadiene, isoprene, 1,3-pentadiene, 2,4-hexadiene and 2,3-dimethylbutadiene-1,3; 1,3-butadiene is a preferred conjugated alkadiene.

The nature of the main reaction product which may be prepared by the process of the present invention will generally comprise an adduct of the active hydrogen-containing compound and a triene which compound can considered to have been obtained by dimerization of the relevant conjugated diene. Hence the reaction product will for example be an alcohol when water has been used as the active hydrogen containing compound, or a secondary or tertiary amine when respectively a primary or secondary amine is used. In general, the hereinbefore described reaction products will be present as admixtures with minor amounts of the corresponding triene.

In the process of the present invention the diphosphines are preferably used in a quantity of from 0.2 to 10 and in particular from 0.5 to 5 mol per gram atom of palladium. The palladium compound will generally be employed in an amount which corresponds with a ratio in the range of from 10⁻⁶ to 10⁻¹ and preferably from 10⁻⁵ to 10⁻² gram atom of palladium per mol of conjugated alkadiene.

The process may conveniently be conducted in the presence of one or more inert solvents and or diluents. Suitable solvents and/or diluents include ketones, such as acetone; ethers, such as dioxane, tetrahydrofuran, anisole (methyl phenyl ether) and diglyme (dimethyl ether of diethylene glycol); hydrocarbon compounds, such as benzene, toluene, cyclohexane and n-hexane; and polar compounds such as dimethylformamide and dimethylsulfoxide. Occasionally water may also be used as a solvent or diluent.

When employing water as the active hydrogen-containing compound in the process of the present invention very good results having been obtained when CO₂ is present in an amount which corresponds with a CO₂ pressure in the range of from 500 to 5000 kPa.

When in the process of the present invention a primary or secondary amine is used as the active hydrogen-containing compound, the diphosphine is a bis(dialkylphosphino) based diphosphine and especially a bis(di-n-butylphosphino) or bis(diisopropylphosphino) based diphosphine. On the other hand when water is employed as the active hydrogen-containing compound, both bis(diarylphosphino) and bis(dialkylphosphino) based diphosphines have resulted in a high conjugated diene conversion and a high selectivity to the linear alcohol type reaction product.

The process of the present invention will generally be conducted at a temperature in the range of from 20 to 180 °C and preferably in a range of from 50 to 150 °C.

The pressure at which the present invention is conducted will generally be in the range of from atmospheric pressure to 10000 kPa, taking in account of course the hereinbefore mentioned preferred presence of carbondioxide when employing water as the active hydrogen-containing compound.

The telomerization products prepared can be recovered for example via fractionation, distillation and/or crystallization, and may advantageously be employed as such or used for example for the synthesis of polymers, synthetic resins, epoxy compounds and surface-active agents.

When water is the active hydrogen-containing compound, the telomerization product (dienol) can be hydrogenated to the corresponding alcohol and subsequently dehydrated to the corresponding alkene. For example, product 3,7-diene-octanol can be hydrogenated to octanol and subsequently dehydrated to 1-octene.

The invention will be further illustrated with the following examples which should however not be considered as limiting the scope of the invention or the manner wherein it may be practised.

The following information is provided for the examples and comparative experiments:

| Di- and monophosphines used in Tables I and II | |
|---|---|
| Code | Phosphine type |
| a) | 1,3-bis(di-n-butylphosphino)propane |
| b) | 1,3-bis(diphenylphosphino)propane |
| c) | 1,2-bis(diphenylphosphino)ethane |
| d) | 1,3-bis(diisopropylphosphino)propane |
| e) | 1,4-bis(diphenylphosphino)butane |
| f) | 1,4-bis(di-n-butylphosphino)butane |
| g) | triphenylphosphine |

| Amines used | |
|---|---|
| p) | n-butylamine |
| q) | diethylamine |

### Example I

Into a 250 ml stainless steel (Hastelloy C) autoclave equipped with a magnetic stirrer were introduced 0.25 mmol Pd(OAc)₂, 0.37 mmol 1,3-bis(di-n-butylphosphino)propane (a), 40 ml anisole and 10 ml n-butylamine. Subsequently the reactor was closed and the air evacuated therefrom, whereupon 10 ml 1,3-butadiene were introduced, and the reactor contents heated to 125 °C, which temperature was maintained for 5 hours. Subsequently the reactor contents were cooled to 20 °C. Analysis by gas liquid chromatography (g.l.c.) indicated that 56% of the butadiene had been converted to a mixture of N-(octadienyl-2,7)-n-butylamine(53%), N,N-di-(octadienyl-2,7)-n-butylamine(29%) and n-octatriene-1,3,7(24%).

### Comparative examples II and III and examples IV and V

The procedure of Example I was repeated with the exception of a change in diphosphine and/or a replacement of the 10 ml of n-butylamine with 15 ml of diethylamine and/or a change in the reaction temperature, as has been indicated in Table I, which Table also presents the product data obtained.

### Comparative experiments A and B

The procedure of Example I was repeated with the exception that 1,3-bis(diphenylphosphino)ethane (c) was used as ligand, while in experiment A the reaction temperature was 135 °C and in experiment B 15 ml of diethylamine were employed. The process details and product data have been included in Table I.

**Table I**

| Example | Phosphine type | Amine type | Temp °C | Butadiene conversion % | Product composition | | |
|---|---|---|---|---|---|---|---|
| | | | | | sec.amine % | tert.amine % | triene % |
| I | a | p | 125 | 56 | 53 | 29 | 24 |
| II (comp.) | b | p | 125 | 25 | 50 | 12 | 30 |
| III (comp.) | b | q | 135 | 25 | - | 90 | 8 |
| IV | a | q | 100 | 78 | - | 95 | 5 |
| V | d | q | 100 | 93 | - | 92 | 7 |

| Comp. exp. | | | | | | | |
|---|---|---|---|---|---|---|---|
| A | c | p | 135 | 20 | 50 | 20 | 25 |
| B | c | q | 125 | < 5 | - | traces (∼1:1) | |

### Examples VI-X

To an autoclave of the type as described in Example I was added, 0.25 mmol Pd(OAc)₂, 0.37 mmol of a diphosphine as indicated in Table II hereinafter, 5 ml water and 40 ml diglyme.

After closing the reactor and evacuating the air therefrom, 10 ml of 1,3-butadiene were added which was followed by pressurizing the reactor with carbondioxide to a pressure corresponding with the value indicated in Table II. The reactor contents were heated to 110 ^{o}C and maintained at this temperature for 5 hours. Subsequently the reaction contents were cooled to 20 ^{o}C and analyzed via g.l.c. Reaction details and product data have been collected in Table II.

### Comparative experiments C-E

The procedure of Examples V-X was repeated with the exception that 1,2-bis(diphenylphosphino)ethane (c) (0,37 mmol) or triphenylphosphine (g) (0,7 mmol) was used as the phosphine compound. Process details and the corresponding product data have been included in Table II.

From the results obtained in Examples I to V (Table I) and those obtained with the comparative experiments A and B, it can be observed that the use of diphosphines having at least 3 atoms in the bridge as described hereinbefore, and especially the bis(dialkylphosphino) based diphosphines can result in very high conjugated diene conversions in combination with a high selectivity to the telomerized products.

A similar phenomenon can be observed when comparing the results of Examples VI-X (Table II) with those of comparative experiments C and E.

Finally it can be concluded that as the catalyst systems of the process of the present invention are based on chelating ligands, they will be more stable than the corresponding systems based on monophosphines.

**Table II**

| Example | Phosphine type | CO₂ pressure kPa | Temp °C | Butadiene conversion % | Product composition | | |
|---|---|---|---|---|---|---|---|
| | | | | | primary alcohol % | secondary alcohol % | triene % |
| VI | a | 1000 | 110 | 88 | 70 | 7 | 22 |
| VII | a | 2000 | 110 | 92* | 80 | 6 | 12 |
| VIII | b | 1000 | 110 | 85 | 71 | 7 | 20 |
| IX | e | 1000 | 110 | 92 | 67 | 6 | 25 |
| X | f | 1000 | 110 | 90 | 61 | 6 | 30 |

| Comp. exp. | | | | | | | |
|---|---|---|---|---|---|---|---|
| C | c | 2000 | 110 | 5 | ∼50 | ∼5 | ∼45 |
| D | g | 2000 | 110 | 95 | 55 | 12 | 30 |
| E | g | 1000 | 110 | 85 | 49 | 9 | 40 |

| | | | | | | | |
|---|---|---|---|---|---|---|---|
| * The reaction medium included 10 ml triethyl amine | | | | | | | |

## Claims

1. A process for the telomerization of conjugated alkadienes which comprises contacting a conjugated alkadiene with a compound having at least one active hydrogen atom selected from the group consisting of water and primary or secondary amines, in the presence of a catalyst obtainable from a palladium compound and a diphosphine of general formula R¹R²PRPR³R⁴, wherein R is a bivalent organic bridging group containing at least 3 atoms in the bridge, of which at least two are carbon atoms; R¹,R²,R³ and R⁴ are the same or different and represent optionally polar substituted hydrocarbyl groups, with the proviso that when the compound having active hydrogen is water the reaction is conducted in the presence of carbon dioxide and when the compound having active hydrogen is a primary or secondary amine the diphosphine is a bis(dialkylphosphino) based diphosphine and preferably a bis(di-n-butylphosphino) or a bis(diisopropylphosphino) based diphosphine..

2. A process as claimed in claim 1, wherein the bridging group R is an alkylene group containing from 3 to 5 carbon atoms in the bridge.

3. A process as claimed in claim 1 or 2, wherein R¹,R²,R³ and R⁴ are the same and represent an aryl group or an alkyl group having up to 10 carbon atoms and preferably from 2 to 5 carbon atoms.

4. A process as claimed in any one of claims 1 to 3, wherein the diphosphine is selected from the group consisting of 1,3-bis(diphenylphosphino)propane, 1,4-bis(diphenylphosphino)butane, 1,3-bis(diisopropylphosphino)propane, 1,3-bis(di-n-butylphosphino)propane and 1,4-bis(di-n-butylphosphino)butane.

5. A process as claimed in any one of claims 1 to 4, wherein the palladium compound is a palladium carboxylate and preferably palladium acetate.

6. A process as claimed in any one of claims 1 to 5, wherein the diphosphine is present in a ratio of from 0.2 to 10 and preferably in a ratio from 0.5 to 5 mol per gram atom of palladium.

7. A process as claimed in any one of claims 1 to 6, wherein the palladium compound is employed in a ratio in the range of from 10⁻⁶ to 10⁻¹ and preferably from 10⁻⁵ to 10⁻² gram atom of palladium per mol of conjugated alkadiene.

8. A process as claimed in any one of claims 1 to 7, wherein the alkadiene is 1,3-butadiene.

9. A process as claimed in any one of claims 1 to 9, which is conducted at a temperature in the range of from 20 to 180 °C and preferably in a range of from 50 to 150 °C, and at a pressure in the range of from atmospheric pressure to 10000 kPa.

## Patentansprüche

1. Verfahren zur Telomerisation von konjugierten Alkadienen, welches ein Inkontaktbringen eines konjugierten Alkadiens mit einer Verbindung umfaßt, die wenigstens ein aktives Wasserstoffatom aufweist, das aus der aus Wasser und primären oder sekundären Aminen bestehenden Gruppe ausgewählt ist, in Gegenwart eines Katalysators, erhältlich aus einer Palladiumverbindung und einem Diphosphin der allgemeinen Formel R¹R²PRPR³R⁴, worin R eine zweiwertige organische Brückengruppe mit einem Gehalt an wenigstens 3 Atomen in der Brücke ist, wovon wenigstens zwei Kohlenstoffatome sind; R¹,R²,R³ und R⁴ gleich oder verschieden sind und für gegebenenfalls polar substituierte Hydrocarbylgruppen stehen, mit der Maßgabe, daß dann, wenn die Verbindung mit aktivem Wasserstoff Wasser ist, die Reaktion in Gegenwart von Kohlendioxid durchgeführt wird und dann, wenn die Verbindung mit aktivem Wasserstoff ein primäres oder sekundäres Amin ist, das Diphosphin ein Diphosphin auf Bis(dialkylphosphino)-Basis und vorzugsweise ein Diphosphin auf Bis(di-n-butylphosphino)- oder Bis(diisopropylphosphino)-Basis ist.

2. Verfahren nach Anspruch 1, worin die Brückengruppe R eine Alkylengruppe mit einem Gehalt an 3 bis 5 Kohlenstoffatomen in der Brücke ist.

3. Verfahren nach Anspruch 1 oder 2, worin R¹, R², R³ und R⁴ gleich sind und eine Arylgruppe oder eine Alkylgruppe mit bis zu 10 Kohlenstoffatomen und vorzugsweise 2 bis 5 Kohlenstoffatomen darstellen.

4. Verfahren nach einem der Ansprüche 1 bis 3, worin das Diphosphin aus der aus 1,3-Bis(diphenylphosphino)propan, 1,4-Bis(diphenylphosphino)butan, 1,3-Bis(diisopropylphosphino)propan, 1,3-Bis(di-n-butylphosphino)propan und 1,4-Bis(di-n-butylphosphino)butan bestehenden Gruppe ausgewählt ist.

5. Verfahren nach einem der Ansprüche 1 bis 4, worin die Palladiumverbindung ein Palladiumcarboxylat und vorzugsweise Palladiumacetat ist.

6. Verfahren nach einem der Ansprüche 1 bis 5, worin das Diphosphin in einem Verhältnis von 0,2 bis 10 und vorzugsweise in einem Verhältnis von 0,5 bis 5 Mol pro Grammatom Palladium vorhanden ist.

7. Verfahren nach einem der Ansprüche 1 bis 6, worin die Palladiumverbindung in einem Verhältnis im Bereich von 10⁻⁶ bis 10⁻¹ und vorzugsweise 10⁻⁵ bis 10⁻² Grammatom Palladium pro Mol konjugiertes Alkadien verwendet wird.

8. Verfahren nach einem der Ansprüche 1 bis 7, worin das Alkadien 1,3-Butadien ist.

9. Verfahren nach einem der Ansprüche 1 bis 8, welches bei einer Temperatur im Bereich von 20 bis 180°C und vorzugsweise in einem Bereich von 50 bis 150°C und bei einem Druck im Bereich von Atmosphärendruck bis 10000 kPa durchgeführt wird.

## Revendications

1. Procédé de télomérisation d'alcadiènes conjugués, caractérisé en ce que l'on met un alcadiène conjugué en contact avec un composé comportant au moins un atome d'hydrogène actif, choisi dans le groupe constitué par l'eau et des amines primaires ou secondaires, en présence d'un catalyseur que l'on peut obtenir à partir d'un composé du palladium et d'une diphosphine de la formule générale R¹R²PRPR³R⁴, dans laquelle R représente un groupe de pontage organique bivalent contenant au moins 3 atomes dans le pont, dont au moins deux sont des atomes de carbone; R¹, R², R³ et R⁴ sont identiques ou différents et représentent des radicaux hydrocarbyle à substitution polaire éventuelle, avec la condition que lorsque le composé possédant de l'hydrogène actif est l'eau, on entreprenne la réaction en présence de dioxyde de carbone et que lorsque le composé possédant de l'hydrogène actif est une amine primaire ou secondaire, la diphosphine soit une bis-(dialkylphosphino)-diphosphine et, de préférence, une bis-(di-n-butylphosphino)- ou une bis-(diisopropylphosphino)-diphosphine.

2. Procédé suivant la revendication 1, caractérisé en ce que le groupe de pontage R est un radical alkylène contenant de 3 à 5 atomes de carbone dans le pont.

3. Procédé suivant la revendication 1 ou 2, caractérisé en ce que R¹, R², R³ et R⁴ sont identiques et représentent chacun un groupe aryle ou un groupe alkyle comportant jusqu'à 10 atomes de carbone et, de préférence, 2 à 5 atomes de carbone.

4. Procédé suivant l'une quelconque des revendications 1 à 3, caractérisé en ce que l'on choisit la diphosphine dans le groupe formé par les substances qui suivent : 1,3-bis-(diphénylphosphino)-propane, 1,4-bis-(diphénylphosphino)-butane, 1,3-bis-(diisopropylphosphino)-propane, 1,3-bis-(di-n-butylphosphino)-propane et 1,4-bis-(di-n-butylphosphino)-butane.

5. Procédé suivant l'une quelconque des revendications 1 à 4, caractérisé en ce que le composé de palladium est un carboxylate de palladium et, de préférence, l'acétate de palladium.

6. Procédé suivant l'une quelconque des revendications 1 à 5, caractérisé en ce que la diphosphine est présente en un rapport de 0,2 à 10 et, de préférence, en un rapport de 0,5 à 5 moles par atome-gramme de palladium.

7. Procédé suivant l'une quelconque des revendications 1 à 6, caractérisé en ce que l'on utilise le composé du palladium en un rapport qui varie de 10⁻⁶ à 10⁻¹ et, de préférence, de 10⁻⁵ à 10⁻², atome-gramme de palladium par mole d'alcadiène conjugué.

8. Procédé suivant l'une quelconque des revendications 1 à 7, caractérisé en ce que l'alcadiène est le 1,3-butadiène.

9. Procédé suivant l'une quelconque des revendications 1 à 8, caractérisé en ce qu'on l'entreprend à une température de 20 à 180°C et, de préférence, dans la plage de 50 à 150°C, et sous une pression qui fluctue de la pression atmosphérique à 10000 kPa.
